# EUROPEAN PATENT APPLICATION

(11) **EP 3 777 775 A1**
(43) Date of publication of application: **17.02.2021**
(21) Application number: 20188575.3
(22) Date of filing: 30.07.2020
(51) Int. Cl.: A61F 2/30, A61F 2/32, A61F 2/34, A61F 2/36

(54) **FEMORAL RESURFACING PROSTHESIS**

(30) Priority: 13.08.2019 IT 201900014745
(71) Applicant: Melozzi, Alessandro, 64100 Teramo (TE) (IT)
(72) Inventor: Melozzi, Alessandro, 64100 Teramo (TE) (IT)
(74) Representative: Cutropia, Gianluigi

(57) **Abstract**

Femoral resurfacing prosthesis (100) comprising an acetabular cup (2) suitable for being implanted in an iliac bone (5) in such a way to reconstruct a cotyloid cavity, a head (3) suitable for being fixed to a femur (4) in such a way to resurface the femur and suitable for being coupled with the acetabular cup (2) in omnidirectional spherical coupling mode, and a stem (1) fixed to the head and acting as guide of the head for positioning in the femur (4). The stem (1) is separate from the head and is coupled with the head in screw or fit-in coupling mode. The head (3) is made of cross-lined polyethylene or polyether-ether-ketone (PEEK) and the stem (1) is completely made of a material that can be reabsorbed by the femoral bone.

## Description

The present invention relates to a femoral resurfacing prosthesis.

Femoral resurfacing prostheses that comprise two components are known:
- a femoral component suitable for being implanted as resurfacing on a femur; and
- an acetabular component suitable for being implanted in a cotyloid cavity.

The femoral component has a head that is shaped like a spherical cap and coupled in omnidirectional spherical coupling mode inside the acetabular component that is shaped like a cup.

Such resurfacing prostheses are used for very young patients because of low surgical invasiveness and high bone saving, in case of medium-long term reviews.

However, the resurfacing prostheses that are implanted in patients are impaired by several problems, which are mainly due to the fact that the head of the femoral component and the cup of the acetabular component are made of a metal material. In fact, metallosis is generated in such an instance, and metal ions are released because of the metal-on-metal coupling of the head with the acetabular cup.

In particular, if the head of the resurfacing prosthesis is made of cobalt-chrome superalloy, large quantities of cobalt and chrome ions are found in the blood of patients, with severe health consequences (see J&J Depuy lawsuit).

US2003/163202 discloses a resurfacing prosthesis that comprises a stem coupled with a head in Morse tapered mode. The head is of metal material and is suitable for being coupled with a metal acetabular cup in metal-on-metal frictional mode.

EP2324800 and EP1290992 disclose total prostheses provided with a head that completely replaces the femoral head of the patient; namely, the head of said prostheses does not resurface and is not in contact with the bone of the femoral head of the patient. The total prosthesis is not designed to resurface the bone of the femoral head of the patient, and for this reason total prostheses are substantially different from resurfacing prostheses. Moreover, total prostheses require the insertion of a long metal stem in the femoral canal.

EP2324800 discloses a total prosthesis head comprising a metal substrate of cobalt-chrome and/or titanium and a PEEK or HXPLE polymer covering. The metal substrate of the head is coupled with a metal stem suitable for being permanently implanted in the femoral canal.

EP1290992 discloses a total prosthesis comprising an acetabular cup coated with hydroxyapatite to favor the bone regrowth of the acetabulum. However, it does not provide any precepts on the regrowth of the bone of the femoral head, given that the femoral head must be completely removed. Such a total prosthesis provides for an aluminum stem that is cemented inside the femoral canal.

The purpose of the present invention is to eliminate the drawbacks of the prior art, by disclosing a femoral resurfacing prosthesis that is safe, reliable, efficient, inexpensive and easy to make and implant.

Another purpose of the present invention is to disclose a femoral resurfacing prosthesis that is as little invasive as possible and favors the regrowth of the femoral bone.

These purposes are achieved according to the invention with the characteristics of the independent claim 1.

Advantageous embodiments of the invention appear from the dependent claims.

The femoral resurfacing prosthesis is defined in the independent claim 1.

Additional features of the invention will be manifest from the following description, which refers to a merely illustrative, not limiting embodiment shown in the appended figures, wherein:

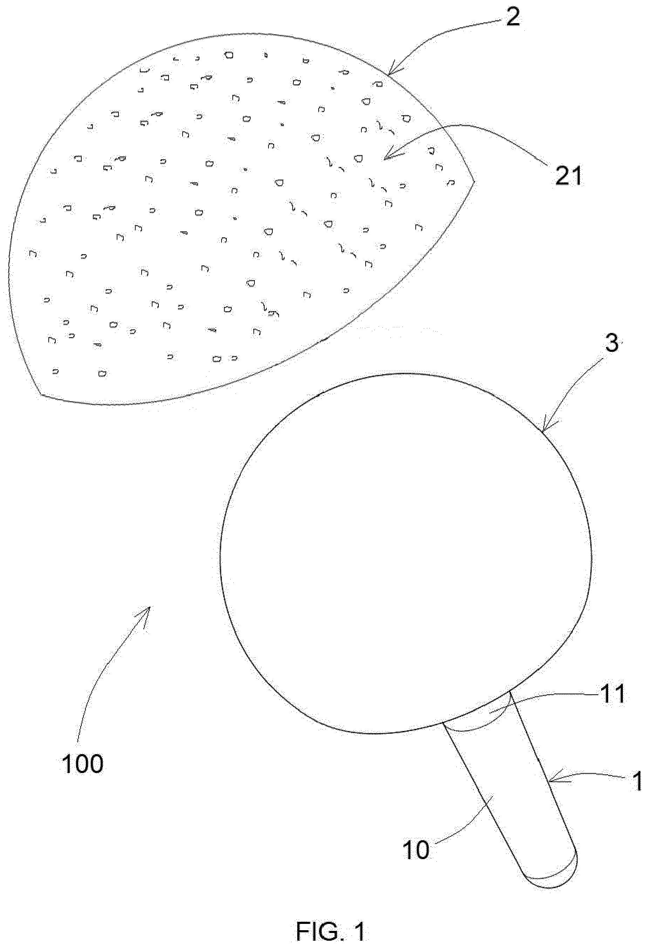

Fig. 1 is a perspective view of a femoral resurfacing prosthesis according to the invention, wherein the acetabular cup is spaced from the head;

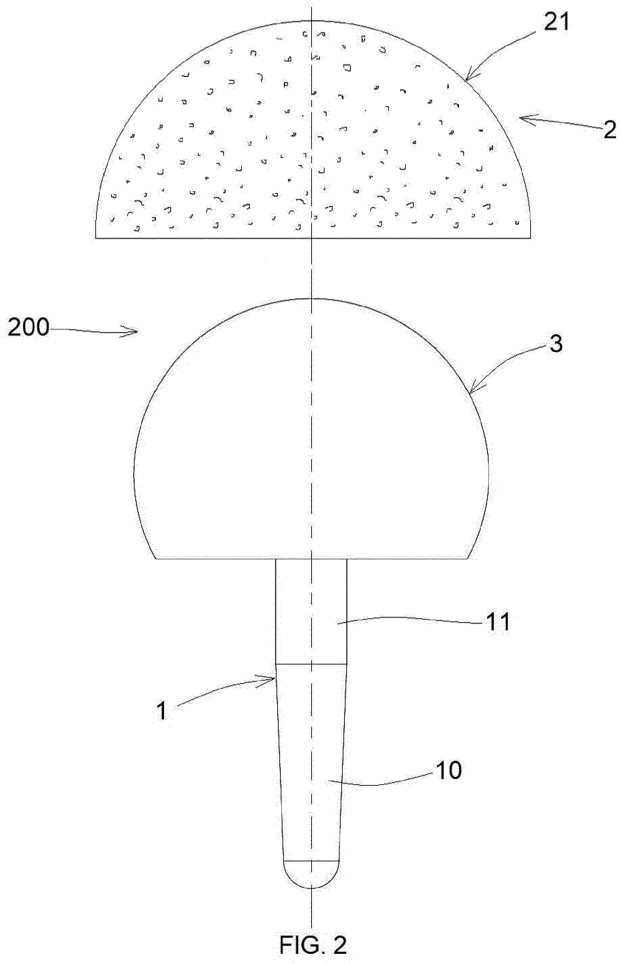

Fig. 2 is a side view of the femoral resurfacing prosthesis of Fig. 1;
Fig. 3 is an axial view of the prosthesis of Fig. 2, wherein the head is coupled inside the acetabular cup;

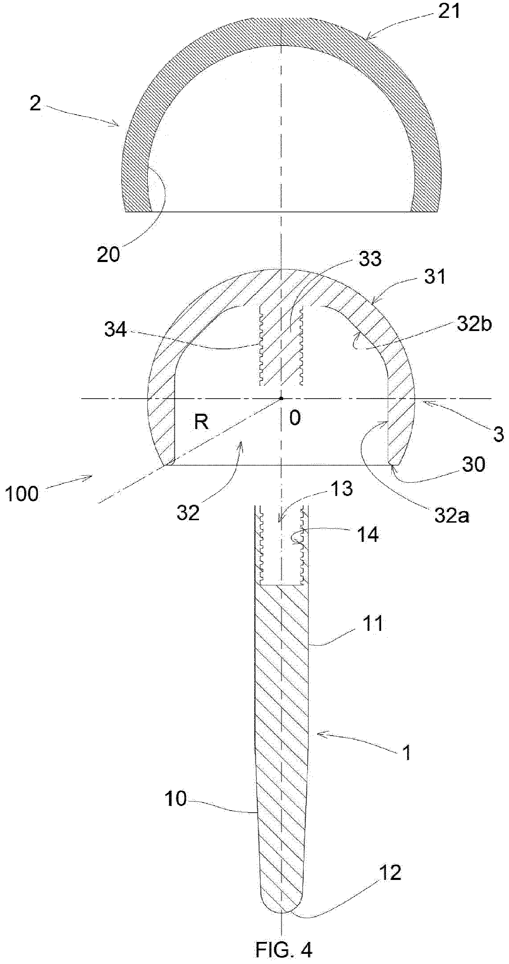

Fig. 4 is an exploded axial view of the three components of the prosthesis of the invention;

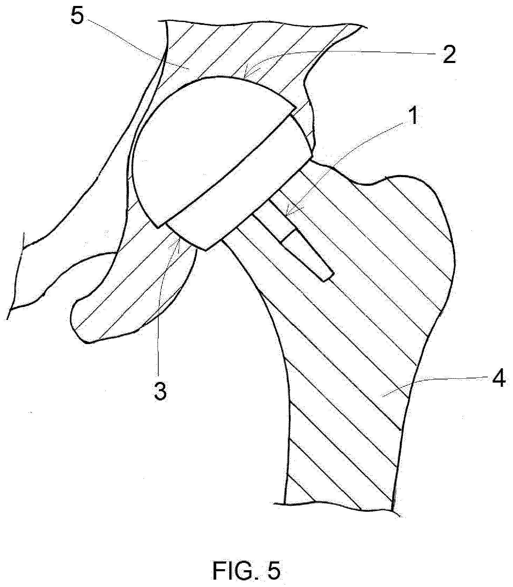

Fig. 5 is a diagrammatic view that shows the femoral resurfacing prosthesis of the invention implanted in a femur of a patient;
Fig. 6 is a perspective axial view of a first variant of the femoral resurfacing prosthesis according to the invention; and
Fig. 7 is a perspective rendering view of a second variant of the femoral resurfacing prosthesis according to the invention.

With reference to the Figures, a femoral resurfacing prosthesis is disclosed, which is generally indicated with reference numeral (100).

The femoral resurfacing prosthesis (100) comprises three components:
- an acetabular cup (2) suitable for being implanted in an iliac bone (5) in such a way to reconstruct a cotyloid cavity;
- a head (3) suitable for being fixed as resurfacing on an upper portion of a femur (4) and suitable for being coupled with the acetabular cup (2) in omnidirectional spherical coupling mode, and
- a stem (1) suitable for being fixed to the head (3) and acting as guide for fixing the head to the femur (4).

The stem (1) has lower dimensions with respect to a stem of a traditional hip prosthesis. In fact, the function of the stem of a traditional hip prosthesis is to be inserted and implanted in the femoral canal in order to act as supporting element for the head of the prosthesis. On the contrary, the stem (1) of the femoral resurfacing prosthesis is not inserted in the femoral canal and only acts as guide for the head. As a matter of fact, the head (2) is fixed, implanted and supported directly by the femur (4) because it resurfaces the femur.

The stem (1) has a lower portion (10) with truncated-conical shape and an upper portion (11) with cylindrical shape. The lower portion (10) ends with a rounded lower end (12).

The upper portion (11) is longer than the lower portion (10).

The stem (1) has a blind hole (13) disposed in axial position and open on top. The blind hole (13) has anchoring means (14) that can consist in an internal thread or a grooved surface with a plurality of annular ribs and grooves.

The stem (1) is made in one piece with a material that can be completely reabsorbed in the femoral bone, such as hydroxyapatite.

The acetabular cup (2) is shaped like a semispherical cap, namely a spherical cap cut at the height of the equator (parallel at 0°).

The acetabular cup (2) has an internal surface (20) with concave shape and an external surface (21) with convex shape suitable for being implanted in the iliac bone of the patient.

The external surface (21) is made of a material that stimulates bone regrowth, such as for example hydroxyapatite. Therefore, the acetabular cup (2) can be of a metal material that is externally coated with hydroxyapatite. Therefore, the internal surface (20) of the acetabular cup is made of metal material.

The head (3) is shaped like a spherical cap cut at the height of a parallel at approximately 20-30° South, in such a way to have a planar base (30). The head (3) has an external surface (31) shaped like a spherical cap with a center (O) and a radius of curvature (R). The external surface (31) of the head is coupled with the internal surface (20) of the acetabular cup, in omnidirectional spherical mode, in such a way that the head (3) can frictionally slide and move with respect to the acetabular cup (2) in any direction around the center (O) of the head.

The head (3) has a channel (32) that is open on the bottom in the base of the head. The channel (32) has higher dimensions than the diameter of the stem (1).

The channel (32) of the head has a cylindrical lower portion (32a) that continues with a tapered upper portion (32b) with decreasing diameter.

The head (3) has a shank (33) that protrudes in lower position in the channel (32). The shank (33) has an external surface provided with anchoring means (34) that are complementary to the anchoring means (14) of the blind hole of the stem. The anchoring means (34) of the shank of the head can be an external thread or a grooved surface provided with a plurality of grooves and ribs. The length of the shank (33) is such that the shank (33) does not reach the center (O) of the head.

The shank (33) of the head is suitable for being coupled inside the blind hole (13) of the stem, in screw or fit-in coupling mode. The anchoring surfaces (34, 14) of the shank (33) and of the hole (13) of the stem are engaged mutually in such a way to prevent an axial extraction of the shank of the head from the hole of the stem.

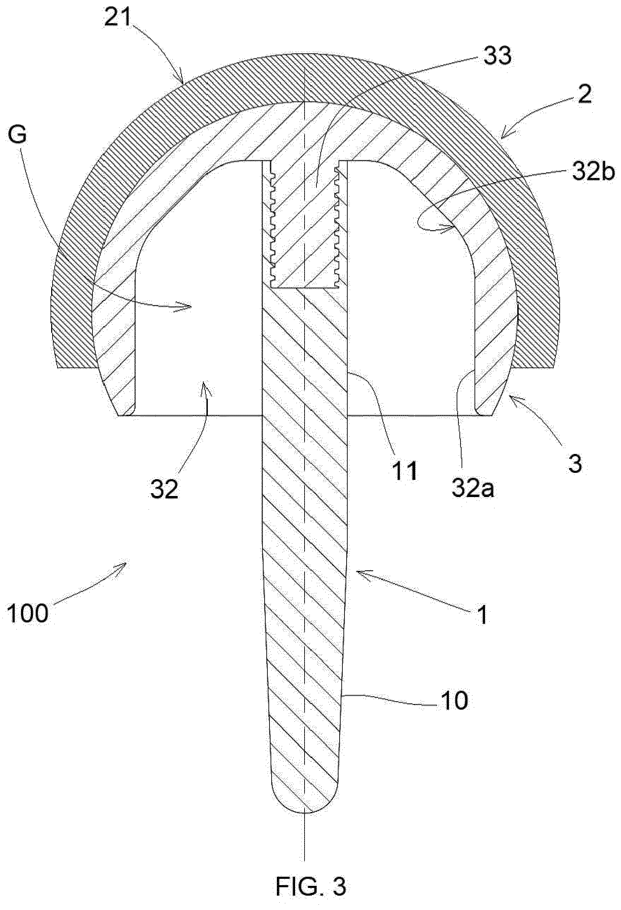

With reference to Fig. 3, when the head (3) is fixed to the stem (1), a hollow space (G) is generated in the channel (32) of the head between the head and the stem. Said hollow space (G) is used for the bone regrowth of the femur (4) inside the head (3), in such a way that the head (3) resurfaces the bone of the femur (4), remaining firmly anchored to the femur (4) and acting as a real femoral head. In such a case, the head of the femoral bone of the patient is milled in such a way to take the same shape as the hollow space (G).

Alternatively, if the head of the femoral bone of the patient cannot be shaped like the hollow space (G) of the resurfacing prosthesis, the hollow space (G) of the resurfacing prosthesis is filled with an acrylic cement that firmly adheres to the bone of the femur (4) of the patient.

The head (3) is made in one piece with cross-linked polyethylene (PEX, XPE or XLPE) o polyether-ether-ketone (PEEK) possibly enriched with carbon fibers.

Advantageously, the head (3) can be made of vitamin E-enriched cross-linked polyethylene.

In view of the above, it is not necessary to change the acetabular cup (2), which can be an acetabular cup of the prior art, or the surgical technique and instruments for the implantation of the prosthesis.

If the hollow space (G) of the femoral resurfacing prosthesis is not to be filled with cement, when a head (3) of cross-linked polyethylene is used - said cross-linked polyethylene being smooth and not suitable for bone anchoring - the internal surface of the channel (32) of the head can be coated with trabecular titanium to favor anchoring and bone regrowth in the trabecula of the titanium coating.

On the contrary, if the head (3) is made of PEEK, which is an intrinsically rough material, a trabecular titanium coating is not necessary and the PEEK can be made rough or a hydroxiapatite coating can be used.

The use of a head made of cross-linked polyethylene or PEEK has several advantages:
- the head (3) of cross-linked polyethylene or PEEK has a natural cartilage-like shock absorber effect, softly distributing the loads on the femur (4),
- the coupling between the metal of the acetabular cup (2) and the cross-linked polyethylene or PEEK of the head (3) guarantees an efficient mutual sliding of the surfaces of the head and of the acetabular cup, avoiding metallosis because of metal-on-metal friction,
- the head (3) of cross-linked polyethylene or PEEK can be produced with injection molding technology, thus considerably simplifying the production process and reducing the production costs compared with traditional metal heads,
- when the hollow space (G) of the resurfacing prosthesis is filled with cement, the head (3) of cross-linked polyethylene or PEEK acts as insulating material, avoiding the problems caused by the high temperature increase for the setting of the cement.

Moreover, it must be considered that the stem (1) is completely made of a material that can be reabsorbed by the bone, such as hydroxyapatite. The use of such a material for the stem provides an effective fit-in coupling with the shank (33) of cross-linked polyethylene or PEEK of the head, and also an effective regrowth and reabsorption of the bone of the femur (4) around the stem and inside the channel (32) of the head.

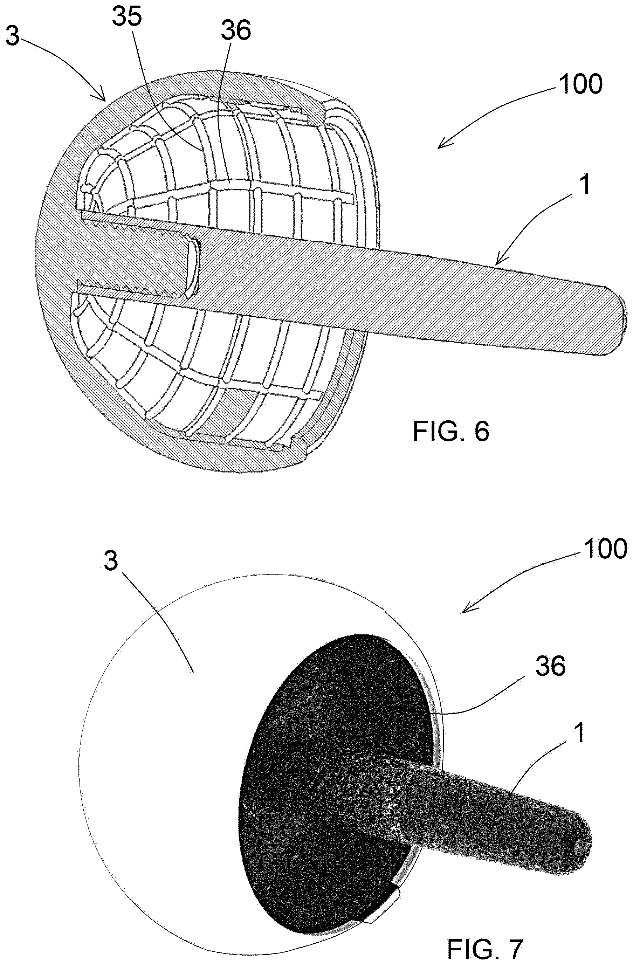

Fig. 6 shows an embodiment wherein the channel (32) of the head (3) has an internal surface provided with a plurality of grooves and/or ribs (35, 36) suitable for providing the anchoring of the cement. For illustrative purposes, a plurality of grooves and/or ribs (35) disposed as parallels and a plurality of grooves and/or ribs (35) disposed as meridians are provided.

Fig. 7 is a rendering view of a resurfacing (36) of the internal surface of the head, with a material that favors anchoring and bone regrowth, such as trabecular titanium, electrowelded titanium, sprayed titanium powder or hydroxyapatite. On the contrary, the stem (1) is completely made of a material that can be reabsorbed by the femoral bone, such as hydroxyapatite.

## Claims

1. Femoral resurfacing prosthesis (100) comprising:
- an acetabular cup (2) suitable for being implanted in an iliac bone (5) in such a way to reconstruct a cotyloid cavity;
- a head (3) suitable for being fixed to a femur (4) in such a way to resurface the femur, without completely removing the femoral head, said head (3) of the prosthesis being suitable for being coupled with the acetabular cup (2), in omnidirectional spherical coupling mode, and
- a stem (1) fixed to the head and acting as guide of the head for positioning in the femur (4); wherein the stem (1) is separate from the head and coupled with the head in screw or fit-in coupling mode,
**characterized in that**
said head (3) is made of cross-linked polyethylene or polyether-ether-ketone (PEEK); and
said stem (1) is completely made of a material that can be reabsorbed by the femoral bone.

2. The femoral resurfacing prosthesis (100) of claim 1, wherein said stem (1) is completely made of hydroxyapatite.

3. The femoral resurfacing prosthesis (100) of claim 1 or 2, wherein the head (3) has a shank (33) that extends in a channel (32) and is coupled with a blind hole (13) that is axially obtained in said stem (11), in such a way that an upper portion (11) of the stem is disposed in said channel (32) of the head, generating a hollow space (G) between the stem and the head.

4. The femoral resurfacing prosthesis (100) of any one of the preceding claims, wherein said head (3) is made of vitamin E-enriched cross-linked polyethylene.

5. The femoral resurfacing prosthesis (100) of any one of the preceding claims, wherein said head (3) is made of polyether-ether-ketone (PEEK) reinforced with carbon fiber.

6. The femoral resurfacing prosthesis (100) of any one of the preceding claims, wherein said head (3) is made in one piece.

7. The femoral resurfacing prosthesis (100) of any one of the preceding claims, wherein said head (3) is injection molded.

8. The femoral resurfacing prosthesis (100) of any one of claims 3 to 7, wherein the internal surface of said channel (32) of the head is coated with a coating (36) of titanium trabecular or hydroxiapatite to favor the anchoring and regrowth of the femoral bone (4) inside said channel (32) of the head.

9. The femoral resurfacing prosthesis (100) of any one of claims 3 to 7, wherein the internal surface of said channel (32) of the head has a plurality of grooves and/or ribs (35, 36) suitable for providing a cement anchoring.

10. The femoral resurfacing prosthesis (100) of claim 9, wherein said plurality of grooves and/or ribs of the internal surface of the channel (32) of the head comprises grooves and/or ribs (35) disposed as parallels and grooves and/or ribs (35) disposed as meridians.

11. The femoral resurfacing prosthesis (100) of any one of the preceding claims, wherein said acetabular cup (2) is made of a metal material coated on the external surface (21) with a material that favors bone regrowth.

12. The femoral resurfacing prosthesis (100) of any one of claims 3 to 11, wherein said shank (33) of the head has an external surface with anchoring means (34) that are engaged with anchoring means (14) provided in said blind hole of the stem.
